# EUROPÄISCHE PATENTSCHRIFT

(11) **EP 0 360 087 B1**
(45) Veröffentlichungstag und Bekanntmachung des Hinweises auf die Patenterteilung: **24.03.1993**
(21) Anmeldenummer: 89116592.0
(22) Anmeldetag: 08.09.1989
(51) Int. Cl.: C07C 69/675, C07C 67/03, C08G 18/06

(54) **Neue Estergruppen gebunden enthaltende Dihydroxyverbindungen, Verfahren zu ihrer Herstellung und ihre Verwendung**
Dihydroxy compounds attached to ester groups, process for their preparation and their use
Composés dihydroxylés rattachés à des groupes esters, procédé pour leur préparation et leur utilisation

(30) Priorität: 17.09.1988 DE 3831681
(43) Veröffentlichungstag der Anmeldung: 28.03.1990
(73) Patentinhaber: BASF Aktiengesellschaft, 67063 Ludwigshafen (DE)
(72) Erfinder: Bott, Kaspar, Dr., D-6800 Mannheim 1 (DE)

(56) Entgegenhaltungen:
- Keine Entgegenhaltungen

## Beschreibung

Erfindungsgegenstand sind Estergruppen gebunden enthaltende Dihydroxyverbindungen der Formel I 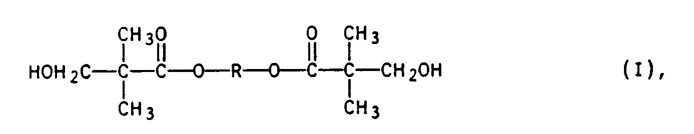
die aufgrund der sterischen Abschirmung der Hydroxylgruppen eine verminderte Reaktivität zeigen sowie ein Verfahren zu ihrer Herstellung und ihre Verwendung in der Polyurethanchemie.

Polyhydroxyverbindungen, beispielsweise höhermolekulare Polyester- oder Polyether-polyole und niedermolekulare mehrwertige Alkohole sind als Aufbaukomponenten zur Herstellung von Polyurethanen von großer technischer Bedeutung. Durch geeignete Wahl dieser Verbindungen unter Berücksichtigung ihrer chemischen Struktur, Funktionalität und Molekulargewichte und in Abhängigkeit von den gewählten Mengenverhältnissen sowie gegebenenfalls anderer Einflußgrößen können in Verbindung mit organischen Polyisocyanaten maßgeschneidert Polyisocyanat-polyadditionsprodukte, wie z.B. Polyurethane, Polyharnstoffe oder Polyisocyanurate mit den verschiedenartigsten mechanischen Eigenschaften hergestellt werden.

Für den Reaktionsablauf von Bedeutung ist, neben z.B. katalytischen oder autokatalytischen Einflüssen, Reaktionstemperatur, Konzentrationsverhältnisse konkurrierender Reaktionspartner, insbesondere die Reaktivität der mit den NCO-Gruppen reaktiven Verbindungen. Diese wird im allgemeinen durch die Basizität der Verbindungen bestimmt und nimmt ab in der Reihenfolge aliphatische Amine, aromatische Amine, aliphatische Harnstoffe, primäre Alkohole, sekundäre Alkohole, Wasser und aromatische Harnstoffe. Beispielsweise verhalten sich die Reaktionsgeschwindigkeiten von primären zu sekundären zu tertiären Alkoholen wie etwa 1 : 0,3 : 0,005. Von Bedeutung für die Reaktivität der Addenden, z.B. der Dihydroxyverbindungen, und die mechanischen Eigenschaften der daraus hergestellten Polyisocyanat-polyadditionsprodukte ist jedoch auch die strukturelle Bindung des reaktiven Rests, beispielsweise ihre räumliche Trennung und/oder gegebenenfalls ihre steriscrhe Abschirmung. Typische Vertreter von Dihydroxyverbindungen mit primären Hydroxylgruppen und verminderter Reaktivität sind 2,2-Dimethyl-propandiol-1.3 und das durch Tischtschenko-Reaktion von Hydroxypivalaldehyd erhältliche 3′-Hydroxy-2′,2′-dimethyl-propyl-3-hydroxy-2,2-dimethyl-propionat. Nachteilig an diesen Dihydroxyverbindungen ist jedoch die geringe Anzahl an Brückenglieder zwischen den reaktiven, sterisch gehinderten Hydroxylgruppen.

Bekannt ist ferner die Herstellung von bei Raumtemperatur flüssigen Diphenylmethan-diisocyanat-Zusammensetzungen.

Nach Angaben der DE-C-16 18 380 (US 3 644 457) werden hierzu ein Mol 4,4′- und/oder 2,4′-Diphenylmethan-diisocyanat - im folgenden kurz MDI genannt - mit 0,1 bis 0,3 Mol Tri-1,2-oxypropylen-glykol und/oder Poly-1,2-oxypropylen-glykol mit einem Molekulargewicht bis 700 zur Reaktion gebracht.

Gemäß GB-A-1 369 334 wird die Modifizierung in zwei Reaktionsstufen durchgeführt und als Modifizierungsmittel Dipropylen-glykol oder Polyoxypropylen-glykol mit einem Molekulargewicht unter 2000 verwendet.

Die DE-A-29 13 126 (US 4 229 347) beschreibt MDI-Zusammensetzungen, in denen 10 bis 35 Gew.% der Isocyanatgruppen mit einem Gemisch aus mindestens 3 Alkylenglykolen umgesetzt werden und wobei eines dieser Glykole Di-, Tri- oder ein höheres Polypropylenglykol ist.

In der DE-A-24 04 166 (GB 1 430 455) werden hingegen als Modifizierungsmittel Gemische aus einem Polyoxyethylen-glykol oder Polyoxyethylen-glykolgemisch mit einem durchschnittlichen Molekulargewicht kleiner als 650 und mindestens einem Alkylenglykol mit mindestens 3 C-Atomen genannt.

Die DE-A 23 46 996 (GB 1 377 679) betrifft MDI-Zusammensetzungen, bei denen 10 bis 35 Gew.% der Isocyanatgruppen mit einem handelsüblichen Polyethylenglykol umgesetzt wurden.

Zur Herstellung von flüssigen Polyisocyanat-Zusammensetzungen wurde neben MDI und Glykolen und/oder Polyoxyalkylenglykolen zusätzlich auch die Mitverwendung von Mischungen aus Diphenylmethan-diisocyanaten und Polyphenyl-polymethylen-polyisocyanaten - im folgenden roh-MDI genannt - beschrieben.

Nach der EP-A-10 850 besteht eine derartige Polyisocyanat-Zusammensetzung aus einem mit Polyoxyalkylen-polyolen mit einer Funktionalität von 2 bis 3 auf Basis von Polyoxypropylen-polyol und gegebenenfalls Polyoxyethylen-polyol mit Molekulargewichten von 750 bis 3000 modifiziertem MDI im Gemisch mit roh-MDI.

Eine flüssige roh-MDI-Zusammensetzung wird nach der DE-B-27 37 338 (US 4 055 548) durch Vereinigen von roh-MDI mit einem Polyoxyethylen-glykol mit einem durchschnittlichen Molekulargewicht von 200 bis 600 erhalten.

Nach Angaben der DE-B-26 24 526 (GB-PS 1 550 325) wird ein nach einem speziellen Verfahren hergestelltes roh-MDI mit 88 bis 95 Gew.% MDI mit Polyoxypropylen-glykol des Molekulargewichtsbereichs 134 bis 700 umgesetzt.

Die DE-A-25 13 796 (GB 1 444 192) und DE-A-25 13 793 (GB 1 450 660) betreffen roh-MDI-Zusammensetzungen, bei denen das roh-MDI mit Alkylen- oder Polyoxyalkylen-glykolen in bestimmten Mengen modifiziert wurde.

Eine flüssige, Urethangruppen enthaltende Polyisocyanatmischung auf MDI-Basis mit einem Gehalt an Isocyanatgruppen von 12 bis 30 Gew.% wird nach Angaben der EP-B-111 121 (US-A-4 478 860) erhalten durch Umsetzung von roh-MDI mit einem Polyoxypropylen-polyoxyethylen-polyol mit einer Funktionalität von 2 bis 4, einer Hydroxylzahl von 10 bis 65 und einem Gehalt an polymerisierten Ethylenoxidgruppen von 5 bis 30 Gew.%, bezogen auf das Gewicht an polymerisierten Ethylenoxid- und Propylenoxidgruppen.

Die genannten Alkylen- oder Polyoxyalkylen-glykole bewirke war eine Verflüssigung der bei 42°C bzw. 28°C schmelzenden 4,4′- bzw. 2,4′-MDI-Isomeren. Nachteilig ist jedoch, daß die Polyisocyanat-Zusammensetzung bei Temperaturen um 10°C nur begrenzt lagerstabil sind und danach kristalline Ausscheidungen zeigen. Diese bewirken eine Inhomogenität der Polyisocyanat-Zusammensetzung und erschweren die maschinelle Verarbeitbarkeit.

Bekannt ist ferner, daß durch die Art der Modifizierung der MDI- oder roh-MDI-Zusammensetzungen die mechanischen Eigenschaften der daraus hergestellten Polyurethane beeinflußt wird, d.h. zur Erzielung einer bestimmten mechanischen Eigenschaft im Polyurethanendprodukt bedarf es der Entwicklung eines neuen spezifischen Modifizierungsmittels.

Die Aufgabe der vorliegenden Erfindung bestand in der Entwicklung von Dihydroxyverbindungen mit räumlich abgeschirmten primären Hydroxylgruppen verminderter Reaktivität, bei welchen die Anzahl der Brückenglieder zwischen den reaktiven Hydroxylgruppen variierbar und größer ist, als bei den vorgenannten 2,2-Dimethyl-propandiol-1.3 und hydroxyl- und methylgruppenhaltigen Propylpropionat.

Diese Aufgabe konnte überraschenderweise gelöst werden mittelsDihydroxyverbindungen, hergestellt durch Umesterung von Hydroxypivalinsäurealkylester mit Alkandiolen, Cycloalkandiolen oder Oxyalkylen-glykolen.

Gegenstände der Erfindung sind somit
Estergrupen gebunden enthaltende Dihydroxyverbindungen der Formel I 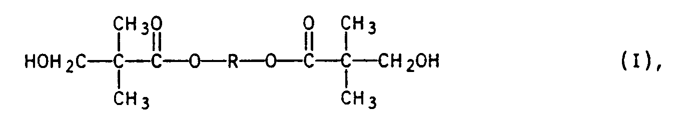
in der R bedeutet:
einen linearen oder verzweigten Alkylenrest mit 2 bis
20 Kohlenstoffatomen,
einen Cycloalkylen- oder substituierten Cycloalkylenrest mit 5 bis
10 Kohlenstoffatomen oder
einen Polyoxyalkylenrest aus 2 bis 50 Oxyalkylengruppen, die angeordnet sind in blockweiser oder statistischer Verteilung und die 2 bis
4 Kohlenstoffatome im Alkylenrest besitzen
und ein Verfahren zur Herstellung der Estergruppen gebunden enthaltenden Dihydroxyverbindungen der Formel (I), das dadurch gekennzeichnet, daß man Hydroxypivalinsäurealkylester mit 1 bis 3 Kohlenstoffatomen im Alkylrest mit linearen oder verzweigten Alkandiolen mit 2 bis 20 Kohlenstoffatomen im Alkylenrest, gegebenenfalls substituierten Cycloalkandiolen mit 5 bis 10 Kohlenstoffatomen im Cycloalkylenrest oder mit Polyoxyalkylen-glykolen mit 2 bis 50 Oxyalkylengruppen, die blockweise oder in statistischer Verteilung gebunden sind und die 2 bis 4 Kohlenstoffatome im Alkylenrest besitzen, umestert.

Gegenstände der Erfindung sind ferner die Verwendung der Estergruppen gebunden enthaltenden Dihydroxyverbindungen der Formel (I) zur Herstellung von bei Raumtemperatur flüssigen, Urethangruppen enthaltenden Polyisocyanatmischungen auf Basis von MDI und roh-MDI gemäß Anspruch 7 und die Verwendung der Dihydroxyverbindungen der Formel (I) als Kettenverlängerungsmittel für Polyisocyanat-polyadditionsreaktionen.

Die erfindungsgemäßen Estergruppen gebunden enthaltenden Dihydroxyverbindungen weisen den Vorteil auf, daß durch geeignete Wahl des Restes R die physikalischen Eigenschaften, wie z.B. die Verträglichkeit, beispielsweise Löslichkeit oder Mischbarkeit, mit anderen Polyurethanaufbaukomponenten, der hydrophile bzw. hydrophobe Charakter, die Flüchtigkeit, die Kristallisationsneigung u.a. der Dihydroxyverbindungen vielfältig variiert und dadurch auch die mechanischen Eigenschaften der Endprodukte gezielt eingestellt werden können, ohne daß die Reaktivität der Hydroxylgruppen nennenswert beeinflußt wird. Durch die sterische Hinderung der Hydroxylgruppen wird, wie bereits ausgeführt wurde, die Reaktivität der primären Hydroxylgruppen reduziert. Dadurch wird jedoch auch der Fließweg von Reaktionsmischungen zur Bildung von zelligen oder kompakten Polyurethan-Elastomerformkörpern oder Schaumstoffen beträchtlich verlängert oder es können größere Formkörper hergestellt werden.

Zu den zur Herstellung der erfindungsgemäßen Dihydroxyverbindungen verwendbaren Ausgangsstoffe und ihrer Verwendung in der Polyurethanchemie ist im einzelnen das folgende auszuführen:
Die erfindungsgemäßen Dihydroxyverbindungen der Formel I können hergestellt werden durch Umesterung von Hydroxypivalinsäurealkylestern mit 1 bis 3 Kohlenstoffatomen, vorzugsweise 1 bis 2 Kohlenstoffatomen im Alkylrest, wie z.B. Hydroxypivalinsäure-n-propyl- oder -isopropylester, vorzugsweise Hydroxypivalinsäureethylester und insbesondere Hydroxypivalinsäuremethylester mit Alkandiolen, Cycloalkandiolen oder Polyoxyalkylen-glykolen in Abwesenheit oder vorzugsweise in Gegenwart von Katalysatoren.

Geeignete Alkandiole besitzen zweckmäßigerweise 2 bis 20 C-Atome, vorzugsweise 2 bis 12 C-Atome im verzweigten oder vorzugsweise linearen Alkylenrest. Beispielhaft genannt seien: 1,2-Ethandiol, 1,2- und 1,3-Propandiol, 2,2-Dimethyl-propandiol-1,3, Butandiol-1,4, 2-Methyl-butandiol-1,4, 2,2-Dimethyl-butandiol-1,4, Pentandiol-1,5, 2-Methyl-pentandiol-1,5, Hexandiol-1,6, 2-Methyl-hexandiol-1,6, 2,2-Dimethyl-hexandiol-1,6, 2,2,4- und 2,4,4-Trimethyl-hexandiol-1,6, 2,2,5,5-Tetramethyl-hexandiol-1,6, Heptandiol-1,7, Octandiol-1,8, 2-Ethyl-octandiol-1,8, Decandiol-1,10, Dodecandiol-1,12 Tetradecandiol-1,14 und Octadecandiol-1,18.

Geeignet sind ferner Cycloalkandiole oder substituierte Cycloalkandiole, wie z.B. mit 1 bis 4 Alkylgruppen, vorzugsweise Methylgruppen, substituierte Cycloalkandiole mit 5 bis 10 C-Atomen, vorzugsweise mit 6 bis 8 C-Atomen im Cycloalkylenrest. In Betracht kommen beispielsweise Cyclopentandiol, 2,3-Dimethyl-, 2,3,5-Trimethyl-, 2,3,5,6-Tetramethyl-cyclohexandiol-1,4 und vorzugsweise die Cyclohexandiol-Isomeren, insbesondere Cyclohexandiol-1,4.

Zur Umesterung besonders bewährt haben sich und daher vorzugsweise Anwendung finden Etherbrücken gebunden enthaltende Glykole aus der Gruppe der Dialkylen-glykole und Polyoxyalkylen-glykole mit 2 bis 50 Oxyalkylengruppen, vorzugsweise 2 bis 20 Oxyalkylengruppen, wobei diese in blockweiser oder statistischer Verteilung gebunden sind und 2 bis 4 Kohlenstoffatome im Alkylenrest besitzen. Als Beispiele seien genannt: Diethylen-, Triethylenglykol und Polyoxyethylenglykole, Dipropylen-, Tripropylenglykol und Polyoxypropylenglykole, Dibutylen-, Tributylenglykol und Polyoxytetramethylenglykole sowie Polyoxyethylen-polyoxypropylen-glykole, Polyoxyethylen-polyoxytetramethylen-glykole und Polyoxypropylen-polyoxytetramethylen-glykole mit blockweiser oder statistischer Verteilung der Oxyalkylengruppen.

Polyoxyalkylen-glykole dieser Art können beispielsweise hergestellt werden durch anionische Polymerisation mit Alkalihydroxiden, wie Natrium- oder Kaliumhydroxid, oder Alkalialkoholaten, wie Natriummethylat, Natrium- oder Kaliumethylat oder Kaliumisopropylat als Katalysatoren und unter Zusatz mindestens eines Startermoleküls, das zwei reaktive Wasserstoffatome gebunden enthält, oder durch kationische Polymerisation mit Lewis-Säuren, wie Antimonpentachlorid, Borfluorid-Etherat u.a. oder Bleicherde als Katalysatoren aus einem oder mehreren Alkylenoxiden mit 2 bis 4 Kohlenstoffatomen im Alkylenrest wie z.B: Tetrahydrofuran, 1,2-Butylenoxid, 1,2-Propylenoxid und Ethylenoxid. Bevorzugt Anwendung finden Polyoxytetramethylenglykole mit Molekulargewichten von 162 bis 1818, vorzugsweise 162 bis 738.

Wie bereits dargelegt wurde, können die Hydroxypivalinsäurealkylester und Alkandiole, Cycloalkandiole oder/und Polyoxyalkylen-glykole katalysatorfrei oder in Gegenwart von Veresterungs- oder vorzugsweise Umesterungskatalysatoren polykondensiert werden. Als Veresterungskatalysatoren kommen beispielsweise Eisen-, Cadmium-, Kobalt-, Blei-, Zink-, Antimon-, Magnesium-, Titan- und Zinnkatalysatoren in Form von Metallen, Metalloxiden oder -salzen, wie z.B. Zinnsalze, z.B. gemäß US 3 162 616, Zinndioctoat und/oder Tetrabutylorthotitanat in Betracht. Als Umesterungskatalysatoren seien beispielhaft genannt: Zinkacetat, Antimonacetat, Tetrabutylorthotitanat und vorzugsweise Alkalialkoholate, wie Natrium- oder Kaliumalkoholat.

Zur Herstellung der erfindungsgemäßen Dihydroxyverbindungen der Formel (I) werden die Hydroxypivalinsäurealkylester und Alkandiole, gegebenenfalls substituierten Cycloalkandiole und/oder vorzugsweise Polyoxyalkylen-glykole im Molverhältnis von 2 : 1 bis 3 : 1, vorzugsweise von 2 : 1 bis 2,5 : 1 verdünnungsmittelfrei oder in Gegenwart von Verdünnungsmitteln sowie katalysatorfrei oder vorzugsweise unter Zusatz von Umesterungskatalysatoren bei Temperaturen von 40 bis 150°C, vorzugsweise von 50 bis 120°C umgeestert. Nach beendeter Umesterung, wozu üblicherweise Reaktionszeiten von 0,5 bis 10 Stunden, vorzugsweise 1 bis 5 Stunden erforderlich sind, werden die überschussigen Hydroxypivalinsäurealkylester, gebildeten Alkohole und gegebenenfalls die Verdünnungsmittel vorzugsweise unter vermindertem Druck abdestilliert.

Die Umesterungsreaktion kann, wie bereits ausgeführt wurde, in Gegenwart von Verdünnungsmitteln sowie gegebenenfalls in einer Atmosphäre aus Inertgasen, wie z.B. Stickstoff, Kohlenmonoxid, Helium oder Argon durchgeführt werden. Als Verdünnungsmittel seien beispielhaft genannt: Benzol, Toluol, Xylol, Chlorbenzol und Dichlorbenzol.

Die nach dem oben beschriebenen Verfahren hergestellten erfindungsgemäßen Estergruppen gebunden enthaltenden Dihydroxyverbindungen der Formel (I) 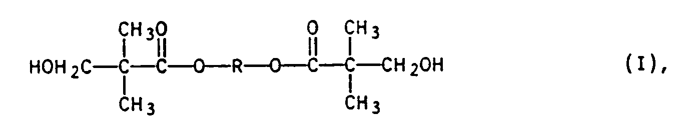
enthalten als Rest -R- vorzugsweise einen 1,2-Ethylen-, 1,2-, 1,3-Propylen-, 2,2-Dimethyl-propylen-1,3-, Butylen-1,4-, 2-Methyl-butylen-1,4-, 2,2-Dimethyl-butylen-1,4-, Pentamethylen-1,5-, 2-Methyl-pentamethylen-1,5-, Hexamethylen-1,6-, 2-Methyl-hexamethylen-1,6-, 2,2-Dimethyl-hexamethylen-1,6-, 2,2,4-, 2,4,4-Trimethyl-hexamethylen-1,6- oder 2,2,5,5-Tetramethyl-hexamethylen-1,6-rest und insbesondere mindestens eine Oxyalkyleneinheit der Formeln

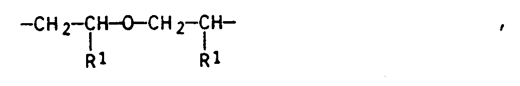

-CH₂-CH₂-CH₂-CH₂-O-CH₂-CH₂-CH₂-CH₂-

oder 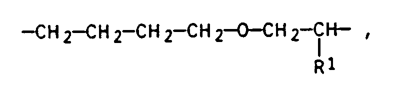
in welchen R¹ gleich oder verschieden ist und Wasserstoff, eine Methyl- oder Ethylgruppe bedeutet.

Die erfindungsgemäßen Dihydroxyverbindungen der Formel I finden vorzugsweise Verwendung zur Herstellung von bei Raumtemperatur flüssigen, Urethangruppen enthaltenden Polyisocyanatmischungen aus 4,4′-Diphenylmethan-diisocyanat oder Gemischen aus 4,4′-Diphenylmethan-diisocyanat und anderen Diphenylmethan-diisocyanat-Isomeren und/oder Polyphenyl-polymethylen-polyisocyanaten. Sie eignen sich ferner als Kettenverlängerungsmittel für Polyisocyanat-polyadditionsreaktionen zur Bildung von beispielsweise kompakten oder zelligen Urethan- oder Urethan- und Harnstoffgruppen enthaltenden Elastomeren sowie als Aufbaukomponente zur Herstellung von hochmolekularen Polyestern oder ungesättigten Polyesterharzen.

Die Herstellung der bei Raumtemperatur flüssigen, Urethangruppen enthaltenden Polyisocyanatmischungen auf 4,4′-MDI-Basis kann nach an sich bekannten Verfahren erfolgen. Nach einer Verfahrensweise wird das roh-MDI, das zweckmäßigerweise einen MDI-Gehalt von 40 bis 95 Gew.%, vorzugsweise 60 bis 90 Gew.% bezogen auf das Gesamtgewicht besitzt, bei Temperaturen von 10 bis 100°C, vorzugsweise von 30 bis 80°C mit einer solchen Menge der erfindungsgemäßen Dihydroxyverbindung der Formel (I) zur Reaktion gebracht, daß das NCO- : OH-Gruppenverhältnis 1 : 0,001 bis 0,30, vorzugsweise 1 : 0,01 bis 0,19 und insbesondere 1 : 0,01 bis 0,1 beträgt. Nach einer Reaktionszeit von 0,5 bis 6 Stunden, vorzugsweise von 1 bis 3 Stunden läßt man die modifizierte Polyisocyanatmischung abkühlen.

Werden die Urethangruppen enthaltenden Polyisocyanatmischungen vorwiegend zur Herstellung von Polyurethan-Weichschaumstoffen verwendet, so wird zweckmäßigerweise nach folgender Verfahrensvariante verfahren: Das 4,4′-MDI oder vorzugsweise eine MDI-Isomerenmischung, die 46 bis 96 Gew.%, vorzugsweise 55 bis 85 Gew.% 4,4′-MDI, 54 bis 2 Gew.%, vorzugsweise 45 bis 14 Gew.%. 2,4-MDI und 0 bis 2 Gew.%, vorzugsweise 0 bis 1 Gew.% 2,2′-MDI bezogen auf das Gesamtgewicht enthält, und die erfindungsgemäße Dihydroxyverbindung der Formel (I) wird bei Temperaturen von 20 bis 100°C, vorzugsweise 40 bis 80°C in einer solchen Menge zur Reaktion gebracht, daß das Verhältnis von NCO- : OH-Gruppen 1 : 0,001 bis 0,3, vorzugsweise 1 : 0,01 bis 0,2 und insbesondere 1 : 0,02 bis 0,15 beträgt. Nach einer Reaktionszeit von 0,5 bis 6 Stunden, vorzugsweise von 1 bis 3 Stunden, läßt man das urethangruppenhaltige MDI auf Temperaturen von 20 bis 80°C abkühlen und verdünnt es mit 4,4′-MDI, der vorgenannten MDI-Isomerenmischung oder vorzugsweise einem roh-MDI mit einem MDI-Gehalt von 45 bis 80 Gew.%, vorzugsweise 50 bis 70 Gew.%, bezogen auf das Gesamtgewicht. Bei Verwendung von 40 bis 90 Gew.-Teilen des urethangruppenhaltigen MDI sind hierfür üblicherweise 60 bis 10 Gew.-Teile 4,4′-MDI, MDI-Isomerengemisch oder roh-MDI erforderlich.

Die flüssigen, Urethangruppen enthaltenden MDI oder roh-MDI Mischungen besitzen zweckmäßigerweise einen NCO-Gehalt von 30 bis 12 Gew.%, vorzugsweise 28 bis 22 Gew.%, bezogen auf das Gesamtgewicht und finden Verwendung zur Herstellung von beispielsweise flexiblen Polyurethanschaumstoffen, Überzugsmassen oder Sportplatzbelägen.

### Beispiele

Herstellung von Estergruppen gebunden enthaltenden Dihydroxyverbindungen.

### Beispiel 1

In einem 2 l-Rührkolben mit aufgesetzter Füllkörperkolonne (20 cm Füllhöhe, 5 mm Stahl-Netze) wurde eine Mischung aus
- 1 300 g: (2.00 Mol) Polyoxytetramethylen-glykol mit einem Molekulargewicht von 650 (OH-Zahl 173),
- 528 g: (4.00 Mol) Hydroxypivalinsäuremethylester mit einer Reinheit von 99 Gew.%
und
- 18,8g: Kaliummethylat

15 min auf 65°C erhitzt. Danach wurde unter vermindertem Druck (300 mbar) das entstandene Methanol am Kopf der Kolonne abdestilliert.

Zur vollständigen Abtrennung des gebildeten Methanols wurde die Temperatur der Reaktionsmischung langsam von 65°C auf 105°C erhöht und gleichzeitig der Druck auf 30 mbar reduziert. Die Umesterung wurde anschließend bei 105°C und unter einem reduzierten Druck von 0,3 mbar in 4 Stunden zu Ende geführt.

Die Entfernung des Kaliumsalzkatalysators erfolgte durch zweistufiges Ausrühren des Umesterungsprodukts mit jeweils 55 g Ambosol® der Firma Hoechst France bei 100°C und Abfiltrieren des Hilfsmittels.

Die auf diese Weise hergestellte Dihydroxyverbindung besaß eine Hydroxylzahl von 127 und einen Kaliumgehalt von 0,0003 Gew.%. Die spektroskopischen Eigenschaften stehen mit der Strukturformel I für R gleich 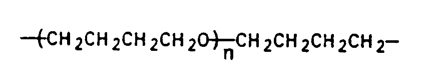
im Einklang.

Isoliert wurden 145 g Destillat, das zu 95 Gew.% aus Methanol und zu 5 Gew.% aus Hydroxypivalinsäuremethylester bestand.

### Beispiel 2

Eine Mischung aus
- 450 g: (0,75 Mol) Polyoxypropylen-glykol mit einem Molekulargewicht von 600,
- 198 g: (1.5 Mol) Hydroxypivalinsäuremethylester und
- 6,7g: Kaliummethylat

wurde in der im Beispiel 1 beschriebenen Apparatur 15 min bei 65°C gerührt.

Das entstandene Methanol wurde unter vermindertem Druck, der zu Beginn der Umesterung 300 mbar und am Ende der Reaktion 1 mbar betrug, abdestilliert. Zur Vervollständigung der Umesterung und Abdestillation des restlichen Methanols wurde die Reaktionsmischung 3 Stunden lang bei 1 mbar auf 100°C erhitzt.

Die Abtrennung des Katalysators erfolgte analog den Angaben des Beispiels 1.

Die erhaltene Dihydroxyverbindung besaß eine Hydroxylzahl von 140 und einen Restkalumgehalt von 0,15 Gew.%. Die spektroskopischen Eigenschaften stehen mit der Strukturformel I für R gleich 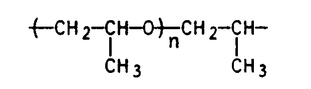
im Einklang.

Herstellung der Urethangruppen enthaltenden Polyisocyanatmischungen auf 4,4′-MDI-Basis

### Beispiel 3

Zu einer Mischung aus
102 Gew.-Teilen 4,4′-MDI und
83 Gew.-Teilen 2,4′-MDI
fügte man unter- Rühren bei 70°C
76,1 Gew.-Teile der Dihydroxyverbindung mit einer Hydroxylzahl von 140,
hergestellt nach den Angaben des Beispiels 2.

Nach einer Nachreaktionszeit von 2 Stunden bei 70°C ließ man das urethangruppenhaltige MDI auf Raumtemperatur abkühlen und verdünnte die Reaktionsmischung mit
227,5 Gew.-Teilen Roh-MDI mit einem NCO-Gehalt von 31 Gew.%.

Die erhaltene Polyisocyanatmischung besaß einen NCO-Gehalt von 25,8 Gew.% und zeigte nach 14tägiger Lagerung bei 3°C keine Sedimentausscheidung.

### Beispiel 4

Zu einer Mischung aus
102 Gew.-Teilen 4,4′-MDI,
83 Gew.-Teilen 2,4′-MDI und
227,5 Gew.-Teilen roh-MDI mit einem Gehalt an MDI-Isomeren von 44 Gew.%
fügte man unter Rühren bei 50°C
76,1 Gew.-Teile der Dihydroxyverbindung mit einer Hydroxylzahl von 140, hergestellt gemäß Beispiel 2
Nach einer Nachreaktionszeit von 2 Stunden bei 70°C ließ man die urethangruppenhaltige Polyisocyanatmischung auf Raumtemperatur abkühlen. Das Produkt besaß einen NCO-Gehalt von 25,9 Gew.% und zeigte nach 14tägiger Lagerung bei -3°C keine Sedimentsausscheidung.

## Patentansprüche

1. Estergruppen gebunden enthaltende Dihydroxyverbindungen der Formel I 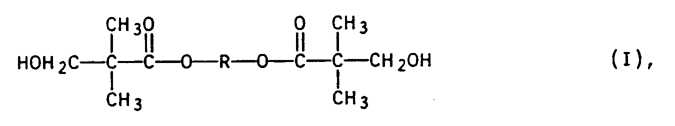 in der R bedeutet:
einen linearen oder verzweigten Alkylenrest mit 2 bis 20 Kohlenstoffatomen, einen gegebenenfalls substituierten Cycloalkylenrest mit 5 bis 10 Kohlenstoffatomen oder einen Polyoxyalkylenrest aus 2 bis 50 Oxyalkylengruppen, die angeordnet sind in blockweiser oder statistischer Verteilung und die 2 bis 4 Kohlenstoffatome im Alkylenrest besitzen.

2. Estergruppen gebunden enthaltende Dihydroxyverbindungen nach Anspruch 1, dadurch gekennzeichnet, daß R einen linearen oder verzweigten Alkylenrest mit 2 bis 12 Kohlenstoffatomen bedeutet, ausgewählt aus der Gruppe 1,2-Ethylen, 1,2-, 1,3-Propylen, 2,2-Dimethylpropylen-1,3, Butylen-1,4, 2-Methyl-butylen-1,4, 2,2-Dimethylbutylen-1,4, Pentamethylen-1,5, 2-Methyl-pentamethylen-1,5, Hexamethylen-1,6, 2-Methyl-hexamethylen-1,6, 2,2-Dimethyl-hexamethylen-1,6, 2,4,4-Trimethylhexamethylen-1,6 und 2,2,5,5-Tetramethyl-hexamethylen-1,6.

3. Estergruppen gebunden enthaltende Dihydroxyverbindungen nach Anspruch 1, dadurch gekennzeichnet, daß R für mindestens eine Oxyalkyleneinheit der Formeln 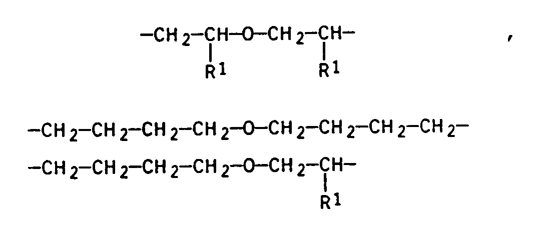 steht, in denen R¹ gleich oder verschieden ist und Wasserstoff, eine Methyl- oder Ethylgruppe bedeutet.

4. Verfahren zur Herstellung von Estergruppen gebunden enthaltenden Dihydroxyverbindungen der Formel (I), dadurch gekennzeichnet, daß man Hydroxypivalinsäurealkylester mit 1 bis 3 Kohlenstoffatomen im Alkylrest mit Alkandiolen mit 2 bis 20 Kohlenstoffatomen im linearen oder verzweigten Alkylenrest, mit Cycloalkan- oder substituierten Cycloalkandiolen mit 5 bis 10 Kohlenstoffatomen im Cycloalkylenrest oder mit Polyoxyalkylen-glykolen mit 2 bis 50 Oxyalkylengruppen, die blockweise oder in statistischer Verteilung gebunden sind und 2 bis 4 Kohlenstoffatome im Alkylenrest besitzen, umestert.

5. Verfahren zur Herstellung von Estergruppen gebunden enthaltenden Dihydroxyverbindungen der Formel (I) gemäß Anspruch 4, dadurch gekennzeichnet, daß man pro Mol Alkandiol, Cycloalkandiol oder Polyoxyalkylen-glykol 2 bis 3 Mole Hydroxypivalinsäurealkylester verwendet.

6. Verfahren zur Herstellung von Estergruppen gebunden enthaltenden Dihydroxyverbindungen der Formel (I) nach einem der Ansprüche 4 oder 5, dadurch gekennzeichnet, daß man die Umesterungsreaktion in Gegenwart von Alkalialkoholaten als Katalysator durchführt.

7. Verwendung von Estergruppen gebunden enthaltenden Dihydroxyverbindungen der Formel (I) zur Herstellung von bei Raumtemperatur flüssigen, Urethangruppen enthaltenden Polyisocyanatmischungen aus 4,4′-Diphenylmethan-diisocyanat oder Gemischen aus 4,4′-Diphenylmethan-diisocyanat und anderen Diphenylmethan-diisocyanat-Isomeren und/oder Polyphenyl-polymethylen-polyisocyanaten

8. Verwendung von Estergruppen gebunden enthaltenden Dihydroxyverbindungen der Formel (I) als Kettenverlängerungsmittel für Polyisocyanat-polyadditionsreaktionen.

## Claims

1. An ester group-containing dihydroxy compound of the formula I 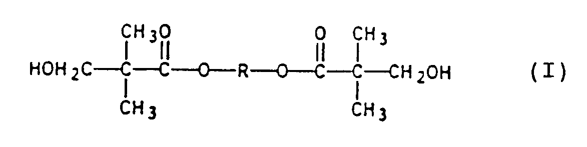 where R is linear or branched alkylene of from 2 to 20 carbon atoms, substituted or unsubstituted cycloalkylene of from 5 to 10 carbon atoms or polyoxyalkylene of from 2 to 50 oxyalkylene groups in a blockwise or random arrangement and having from 2 to 4 carbon atoms in the alkylene radical.

2. An ester group-containing dihydroxy compound as claimed in claim 1, wherein R is linear or branched alkylene of from 2 to 12 carbon atoms selected from the group consisting of 1,2-ethylene, 1,2-propylene, 1,3-propylene, 2,2-dimethyl-1,3-propylene, 1,4-butylene, 2-methyl-1,4-butylene, 2,2-dimethyl-1,4-butylene, 1,5-pentamethylene, 2-methyl-1,5-pentamethylene, 1,6-hexamethylene, 2-methyl-1,6-hexamethylene, 2,2-dimethyl-1,6-hexamethylene, 2,4,4-trimethyl-1,6-hexamethylene and 2,2,5,5-tetramethyl-1,6-hexamethylene.

3. An ester group-containing dihydroxy compound as claimed in claim 1, wherein R is one or more oxyalkylene units of the formulae 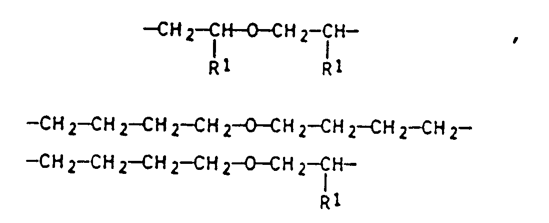 where each R', which may be identical to or different from the others, is hydrogen, methyl or ethyl.

4. A process for preparing an ester group-containing dihydroxy compound of the formula (I), which comprises transesterifying an alkyl hydroxypivalate having from 1 to 3 carbon atoms in the alkyl radical with an alkanediol having from 2 to 20 carbon atoms in the linear or branched alkylene radical, a substituted or unsubstituted cycloalkanediol having from 5 to 10 carbon atoms in the cycloalkylene radical or with a polyoxyalkylene glycol of from 2 to 50 oxyalkylene groups in a blockwise or random arrangement and having from 2 to 4 carbon atoms in the alkylene radical.

5. A process for preparing an ester group-containing dihydroxy compound of the formula (I) as claimed in claim 4, wherein from 2 to 3 moles of alkyl hydroxypivalate are used per mole of alkanediol, cycloalkanediol or polyoxyalkylene glycol.

6. A process for preparing an ester group-containing dihydroxy compound of the formula (I) as claimed in either of claims 4 and 5, wherein the transesterification reaction is carried out in the presence of an alkali metal alcoholate as catalyst.

7. The use of ester group-containing dihydroxy compounds of the formula (I) for preparing urethane group-containing polyisocyanate mixtures that are liquid at room temperature from 4,4'-diphenylmethane diisocyanate or mixtures of 4,4'-diphenylmethane diisocyanate and other diphenylmethane diisocyanate isomers and/or polyphenylpolymethylene polyisocyanates.

8. The use of ester group-containing dihydroxy compounds of the formula (I) as chain extenders for polyisocyanate polyaddition reactions.

## Revendications

1. Composés dihydroxylés contenant des radicaux ester liés de la formule I 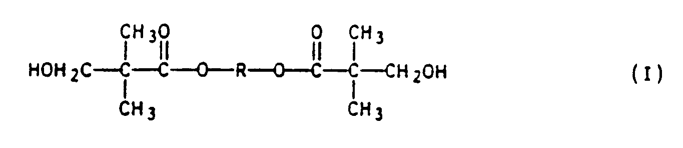 dans laquelle R désigne
un radical alkylène linéaire ou ramifié, qui comporte de 2 à 20 atomes de carbone, un radical cycloalkylène éventuellement substitué, qui comporte de 5 à 10 atomes de carbone, ou un radical polyoxyalkylène, qui comporte de 2 à 50 radicaux oxyéthylène, qui sont agencés à la manière de blocs, ou qui sont en répartition statistique ou stochastique et dont le radical alkylène comporte de 2 à 4 atomes de carbone.

2. Composés dihydroxylés contenant des radicaux ester liés selon la revendication 1, caractérisés en ce que R représente un radical alkylène linéaire ou ramifié, qui comporte de 2 à 12 atomes de carbone, choisi dans le groupe formé par les radicaux qui suivent : 1,2-éthylène, 1,2-, 1,3-propylène, 2,2-diméthylpropylène-1,3, butylène-1,4, 2-méthyl-butylène-1,4, 2,2-diméthylbutylène-1,4, pentaméthylène-1,5, 2-méthyl-pentaméthylène-1,5, hexaméthylène-1,6, 2-méthyl-hexaméthylène-1,6, 2,2-diméthyl-hexaméthylène-1,6, 2,4,4-triméthylhexaméthylène-1,6 et 2,2,5,5-tétraméthyl-hexaméthylène-1,6.

3. Composés dihydroxylés contenant des radicaux ester liés selon la revendication 1, caractérisés en ce que R représente au moins une unité oxyalkylène des formules 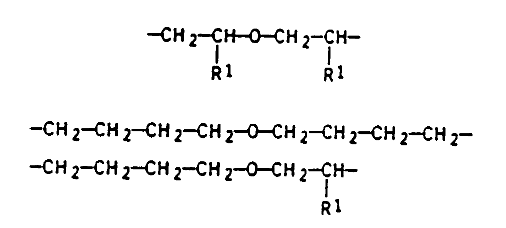 dans lesquelles les symboles R¹, identiques ou différents, représentent chacun un atome d'hydrogène, le radical méthyle ou le radical éthyle.

4. Procédé de préparation de composés dihydroxylés contenant des radicaux ester liés de la formule (I), caractérisé en ce que l'on transestérifie les esters alkyliques de l'acide hydroxypivalique dont le radical alkyle comporte de 1 à 3 atomes de carbone, avec des alcanediols dont le reste alkylène, linéaire ou ramifié, comporte de 2 à 20 atomes de carbone, avec des cycloalcanediols ou des cycloalcanediols substitués, dont le radical cycloalkylène comporte de 5 à 10 atomes de carbone, ou avec des polyoxyalkylèneglycols qui comportent de 2 à 50 radicaux oxyalkylène, qui sont liés à la manière de blocs ou en répartition statistique ou stochastique et dont le radical alkylène comporte de 2 à 4 atomes de carbone.

5. Procédé de préparation de composés dihydroxylés contenant des radicaux ester liés de la formule (I) suivant la revendication 4, caractérisé en ce que l'on utilise de 2 à 3 moles d'ester alkylique de l'acide hydroxypivalique par mole d'alcanediol, de cycloalcanediol ou de polyoxyalkylèneglycol.

6. Procédé de préparation de composés dihydroxylés contenant des radicaux ester liés de la formule (I) suivant l'une des revendications 4 ou 5, caractérisé en ce que l'on entreprend la réaction de transestérification en présence d'alcoolates d'alcalis servant de catalyseurs.

7. Utilisation de composés dihydroxylés contenant des radicaux ester liés de la formule (I) pour la préparation de mélanges à base de polyisocyanates contenant des radicaux uréthanne, liquides à la température ambiante, à partir de diisocyanate de 4,4'-diphénylméthane, ou de mélanges à partir de diisocyanate de 4,4'-diphénylméthane et d'autres isomères du diisocyanate de diphénylméthane et/ou de polyphényl-polyméthylène-polyisocyanates.

8. Utilisation de composés dihydroxylés contenant des radicaux ester liés de la formule (I) à titre d'agents d'allongement des chaînes pour des réactions de polyisocyanatopolyaddition.
